# EUROPEAN PATENT APPLICATION

(11) **EP 4 832 016 A1**
(43) Date of publication of application: **28.10.2026**
(21) Application number: 24906588.9
(22) Date of filing: 23.12.2024
(51) Int. Cl.: G21K 1/02, A61B 6/06

(54) **COLLIMATOR ASSEMBLY, OPTICAL-MECHANICAL ADJUSTMENT APPARATUS, AND X-RAY IMAGING SYSTEM**

(30) Priority: 21.12.2023 CN 202311773943
(71) Applicant: Nuctech Company Limited, TongFang Building Shuangqinglu Haidian District Beijing 100084 (CN); Tsinghua University, Beijing 100084 (CN)
(72) Inventor: CHEN, Zhiqiang, Beijing 100084 (CN); ZHANG, Li, Beijing 100084 (CN); HUANG, Qingping, Beijing 100084 (CN); ZHOU, Lifeng, Beijing 100084 (CN); ZHAO, Xin, Beijing 100084 (CN); CHENG, Yi, Beijing 100084 (CN); HAN, Lina, Beijing 100084 (CN); ZHANG, Linlin, Beijing 100084 (CN); AN, Xuchao, Beijing 100084 (CN)
(74) Representative: Cabinet Beau de Loménie
(86) International application number: PCT/CN2024/141456
(87) International publication number: WO 2025/131118

(57) **Abstract**

A collimator assembly (1) is provided, including: at least one position adjustment portion (11) located at an end portion of the collimator assembly (1) and configured to adjust a position of the collimator assembly (1); a collimator body portion (12), the at least one position adjustment portion (11) is located on the collimator body portion (12), the collimator body portion (12) includes a first adjustment member (121), a left collimator member (122), a right collimator member (123), and adjustment holes (124) provided on the left collimator member (122) and the right collimator member (123), the left collimator member (122) and the right collimator member (123) are arranged side by side with a collimator slit (125) therebetween, each adjustment member (121) penetrates a corresponding adjustment hole (124) to adjust a width of the collimator slit (125); and a shielding box (13) fixed to a side of the collimator body portion (12) opposite to the at least one position adjustment portion (11), and each of a top portion and a bottom portion of the shielding box (13) is provided with a gap (131) corresponding to the collimator slit (125).

## Description

This application claims priority to Chinese Patent Application No. 202311773943.3 filed on December 21, 2023, the entire contents of which are incorporated herein by reference.

### TECHNICAL FIELD

The present disclosure relates to the field of X-ray imaging, and more particularly, to a collimator assembly, an X-ray source adjustment device including the collimator assembly, and an X-ray imaging system including the X-ray source adjustment device.

### BACKGROUND

The background description provided herein is intended to generally present the context of the present disclosure. To the extent described in the background section, work of the presently named inventors, as well as aspects of the description that may not otherwise constitute the prior art at the time of filing, are neither expressly nor implicitly admitted as the prior art against the present disclosure.

An X-ray grading inspection system for pig carcasses differs from conventional X-ray imaging systems. In order to ensure that X-rays can penetrate pork, an X-ray source in such an X-ray imaging system requires larger current and higher voltage. Meanwhile, restricted by site conditions, a distance between an X-ray source target and a collimator is relatively small, leaving limited space for structural design and imposing high pressure on radiation shielding. Therefore, a novel X-ray imaging system is urgently needed.

### SUMMARY

Therefore, the present disclosure provides a novel collimator assembly, an X-ray source adjustment device including the collimator assembly, and an X-ray imaging system, so as to solve the deficiencies in the prior art described above.

According to the present disclosure, a collimator assembly is provided, including: at least one position adjustment portion located at an end portion of the collimator assembly and configured to adjust a position of the collimator assembly; a collimator body portion, the at least one position adjustment portion is located on the collimator body portion, the collimator body portion includes a first adjustment member, a left collimator member, a right collimator member, and adjustment holes provided on the left collimator member and the right collimator member, the left collimator member and the right collimator member are arranged side by side with a collimator slit between the left collimator member and the right collimator member, and the adjustment member penetrates the adjustment hole to adjust a collimator slit width; and a shielding box fixed to a side of the collimator body portion opposite to the at least one position adjustment portion, each of a top portion of the shielding box and a bottom portion of the shielding box is provided with a gap corresponding to the collimator slit.

In an embodiment of the present disclosure, the position adjustment portion includes a boss portion, a through hole is provided in the boss portion, and at least a section of an inner surface of the through hole is provided with a thread.

In an embodiment of the present disclosure, the collimator assembly includes a first boss portion and a second boss portion, each through hole is provided with a second adjustment member having a thread, and the second adjustment member is configured to fix the collimator assembly and adjust a position of the collimator assembly in an axial direction of the through hole.

In an embodiment of the present disclosure, the collimator assembly includes a first boss portion and a second boss portion, and a rotation of the collimator assembly in a plane where the slit width is located is adjustable by rotating corresponding second adjustment mechanisms in the through holes of the first boss portion and the second boss portion by different angles.

In an embodiment of the present disclosure, each of the first adjustment member and the second adjustment member is a screw rod.

In an embodiment of the present disclosure, the collimator slit width is in a range of 0.6 mm to 1.2 mm.

In an embodiment of the present disclosure, each of the at least one position adjustment portion further includes a flat plate portion, the flat plate portion is provided with a plurality of flat plate holes and an elongated gap, the elongated gap is in correspondence with the collimator slit, and the plurality of flat plate holes are in one-to-one correspondence with the adjustment holes provided on the collimator body portion respectively.

In an embodiment of the present disclosure, each of the adjustment holes and each of the plurality of flat plate holes has a smooth inner surface, and the first adjustment member is fixed to the shielding box by penetrating the flat plate hole and the adjustment hole.

In an embodiment of the present disclosure, the left collimator member includes a first protrusion at a position close to the collimator slit, the right collimator member includes a second protrusion at a position close to the collimator slit, and the collimator slit is surrounded by the first protrusion and the second protrusion.

According to the present disclosure, an X-ray source adjustment device is further provided, including: a base, an X-ray tube and an X-ray tube bracket are arranged on the base; an X-ray tube horizontal adjustment member, including a first horizontal adjustment member and a second horizontal adjustment member, the second horizontal adjustment member is located on the X-ray tube bracket, the first horizontal adjustment member is configured to adjust a position of the X-ray tube bracket relative to the base, and the second horizontal adjustment member is configured to adjust a position of the X-ray tube relative to the X-ray tube bracket; and the collimator assembly described in any one of the embodiments above, the collimator assembly is located on the base.

In an embodiment of the present disclosure, the first horizontal adjustment member is arranged on the X-ray tube bracket, the first horizontal adjustment member includes a third adjustment member and a threaded hole, the threaded hole is provided in the X-ray tube bracket, and the third adjustment member is fixed to the base by penetrating the threaded hole.

In an embodiment of the present disclosure, a plurality of second horizontal adjustment members are provided on two sides of the X-ray tube, the second horizontal adjustment member includes a fourth adjustment member and an elongated adjustment hole, the elongated adjustment hole is provided on the X-ray tube bracket, the fourth adjustment member penetrates the elongated adjustment hole to adjust a horizontal position of the X-ray tube relative to the X-ray tube bracket.

In an embodiment of the present disclosure, a longitudinal direction of the elongated adjustment hole is parallel to an axis of the X-ray tube.

In an embodiment of the present disclosure, an axis of the X-ray tube is perpendicular to a slit width direction of the collimator assembly.

In an embodiment of the present disclosure, rays emitted from the X-ray tube penetrate the collimator slit, the slit width is in association with a distance from the collimator assembly to the X-ray tube, and the collimator slit is narrower as the collimator assembly gets closer to the X-ray tube.

In an embodiment of the present disclosure, a vertical adjustment member is further included, the vertical adjustment member includes a screw rod and a nut, the screw rod penetrating the X-ray tube bracket is connected to the base through the nut, and is configured to adjust a vertical position of the X-ray tube relative to the base.

In an embodiment of the present disclosure, the base is arranged in a plane perpendicular to a horizontal plane.

According to the present disclosure, an X-ray imaging system is further provided, including the X-ray source adjustment device described in any one above.

In the present disclosure, the slit width of the collimator is adjustable, so that the collimator may be used in a limited space and may cooperate with the X-ray source to achieve multi-degree-of-freedom adjustment, thereby ultimately achieving alignment of the X-ray source, the collimator, and a detector along a line. Meanwhile, the number of collimator specifications may be reduced, and machining costs may be lowered.

These and other aspects of the present disclosure will become apparent through the following description of the preferred embodiments, taken in conjunction with the accompanying drawings and their descriptions. However, changes and modifications may be made without departing from the spirit and scope of the novel concept of the present disclosure.

### BRIEF DESCRIPTION OF THE DRAWINGS

The present disclosure will be more fully understood through the detailed description and the accompanying drawings. The drawings show one or more embodiments of the present disclosure and, together with the written description, serve to explain the principles of the present disclosure. Wherever possible, the same reference numerals are used throughout the drawings to indicate the same or similar elements in the embodiments, in which:
FIG. 1 is a top view of a collimator assembly according to an exemplary embodiment of the present disclosure;
FIG. 2 is a sectional view taken along line B-B shown in FIG. 1;
FIG. 3 is a sectional view taken along line A-A shown in FIG. 1;
FIG. 4 is a perspective view of a collimator assembly according to an exemplary embodiment of the present disclosure;
FIG. 5 is a perspective view of a collimator assembly mounted on an X-ray source base according to an exemplary embodiment of the present disclosure;
FIG. 6 is a perspective view of an X-ray source adjustment device according to an exemplary embodiment of the present disclosure;
FIG. 7 is a perspective view of an X-ray source adjustment device from another angle according to an exemplary embodiment of the present disclosure;
FIG. 8 is a top view of an X-ray source adjustment device according to an exemplary embodiment of the present disclosure;
FIG. 9 is a sectional view taken along line A-A shown in FIG. 8;
FIG. 10 is a side view of an X-ray source adjustment device according to an exemplary embodiment of the present disclosure; and
FIG. 11 is a perspective view of an X-ray source base, vertically arranged and having an X-ray source adjustment device mounted thereon, according to an exemplary embodiment of the present disclosure.

### DETAILED DESCRIPTION OF EMBODIMENTS

Hereinafter, the present disclosure will be described more fully with reference to the accompanying drawings, in which exemplary embodiments of the present disclosure are shown. However, the present disclosure may be embodied in different forms and should not be construed as being limited to the embodiments described herein. These embodiments of the present disclosure are provided so that the present disclosure will be thorough and complete, and the scope of the present disclosure will be fully conveyed to those skilled in the art. In the drawings, thicknesses of layers and regions may be exaggerated for clarity. Throughout the description, the same reference numerals are used to indicate the same elements. For different embodiments, the elements may have different mutual relationships and different positions.

In the present disclosure, a novel collimator is mainly provided, in which a slit width of the collimator is adjustable, so that the collimator may be used in a limited space and may cooperate with an X-ray source to achieve multi-degree-of-freedom adjustment, thereby ultimately achieving alignment of the X-ray source, the collimator, and a detector along a line. Meanwhile, the number of collimator specifications may be reduced, and machining costs may be lowered.

The collimator assembly in the present disclosure allows for adjustment of the slit width of the collimator, so that the slit width is in a range of 0.6 mm to 1.2 mm. The collimator assembly further allows for adjustment of a position of the collimator on an X-ray source base, so that the collimator cooperates with an X-ray source portion to achieve adjustment and alignment in a certain direction such as a horizontal direction. In addition, the collimator assembly allows for small-angle rotational adjustment of the collimator, for example, a fine adjustment of deflection in a vertical direction. Finally, the X-ray source including the collimator assembly enables more accurate alignment of the X-ray source, the collimator, and the detector along a line by adjusting an X-ray tube base in two directions.

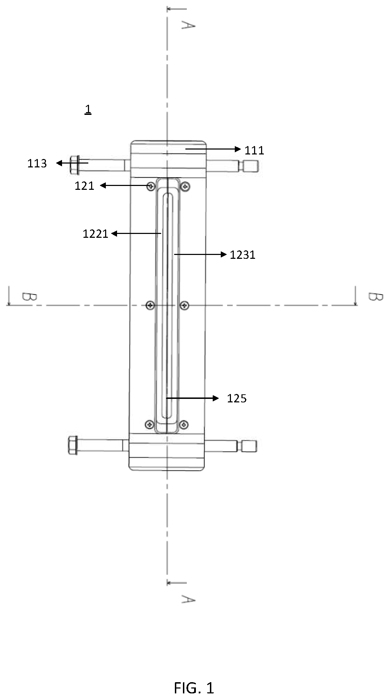

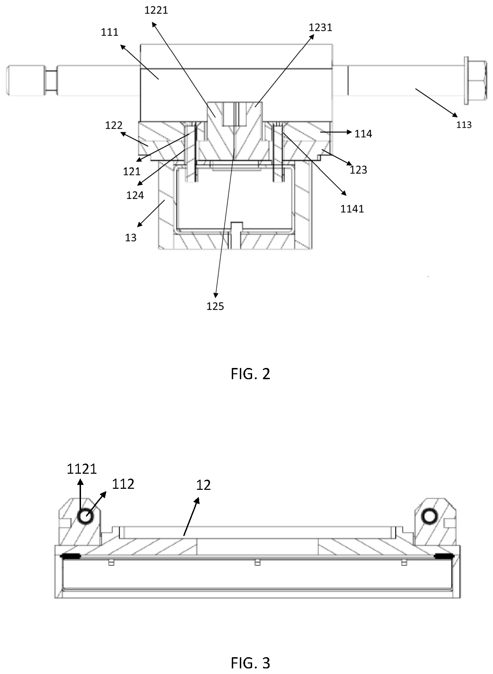

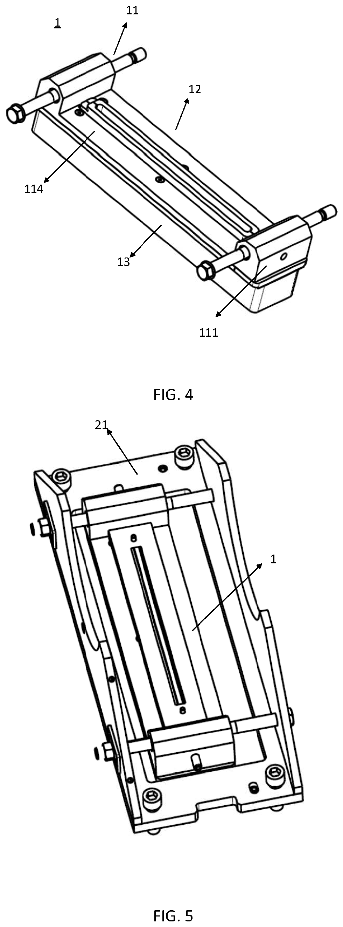

FIG. 1 is a top view of a collimator assembly according to an exemplary embodiment of the present disclosure. FIG. 2 is a sectional view taken along line B-B shown in FIG. 1. FIG. 3 is a sectional view taken along line A-A shown in FIG. 1. FIG. 4 is a perspective view of a collimator assembly according to an exemplary embodiment of the present disclosure. FIG. 5 is a perspective view of a collimator assembly mounted on an X-ray source base according to an exemplary embodiment of the present disclosure.

As shown in FIG. 1 to FIG. 5, in embodiments of the present disclosure, the collimator assembly 1 includes: at least one position adjustment portion 11 disposed at an end portion of the collimator assembly 1 and configured to adjust a position of the collimator assembly 1; a collimator body portion 12, where the at least one position adjustment portion 11 is located on the collimator body portion 12, the collimator body portion 12 includes a first adjustment member 121, a left collimator member 122, a right collimator member 123, and adjustment holes 124 provided on the left collimator member 122 and the right collimator member 123; the left collimator member 122 and the right collimator member 123 are arranged side by side with a collimator slit 125 formed therebetween, and the adjustment member passes through the corresponding adjustment hole 124 to adjust a width of the collimator slit 125; and a shielding box 13 fixed to a side of the collimator body portion 12 opposite to the position adjustment portion 11, where each of a top portion of the shielding box 13 and a bottom portion of the shielding box 13 is provided with a gap 131 corresponding to the collimator slit 125.

In an embodiment of the present disclosure, as shown in FIG. 2 and FIG. 3, the collimator is composed of a plurality of members. For example, the collimator includes a position adjustment portion 11, a collimator body portion 12, and a shielding box 13. The collimator body portion 12 includes a first adjustment member 121, a left collimator member 122, a right collimator member 123, and a plurality of adjustment holes 124 provided on the left collimator member 122 and the right collimator member 123. The first adjustment member 121 may be a screw rod, a bolt, or a screw. A collimator slit 125 is formed between the left collimator member 122 and the right collimator member 123. By adjusting the screws respectively located in the left collimator member 122 and the right collimator member 123, positions of the left collimator member 122 and the right collimator member 123 relative to each other may be adjusted, so that a distance between the left collimator member 122 and the right collimator member 123 may be adjusted. Accordingly, a width of the collimator slit 125 may be adjusted to be in a range of 0.6 mm to 1.2 mm.

In an embodiment of the present disclosure, as shown in FIG. 4 and FIG. 5, the collimator assembly may include one or more position adjustment portions 11 for moving the collimator assembly 1 in a certain direction. Each position adjustment portion 11 includes a boss portion 111. A through hole 112 is provided inside a portion of the boss portion 111 that protrudes from other portions of the collimator, and at least a section of an inner surface of the through hole 112 is provided with a thread 1121. Providing a section of thread rather than a full thread may reduce machining workload, while preventing a threaded engagement portion from being excessively long, thereby facilitating assembly and adjustment. Alternatively, in other embodiments, an entire inner surface of the through hole 112 is provided with a thread.

In an embodiment of the present disclosure, as shown in FIG. 2 to FIG. 5, the collimator assembly 1 may include two position adjustment portions 11, and each position adjustment portion 11 includes a boss portion 111. In other words, the collimator assembly 1 may include a boss portion 111 at each end portion, so that the collimator assembly 1 may move more stably and smoothly in a certain direction. Each through hole 112 is provided with a threaded second adjustment member 113, namely, a screw rod or a bolt. The screw rod or bolt may not only fix the collimator assembly 1, for example, fix the collimator assembly 1 to an X-ray source base 21, but also adjust the position of the collimator assembly 1 in a certain direction, such as the position of the collimator assembly 1 in an axial direction of the through hole 112. Through cooperation between the screw rod or bolt and the thread in the through hole 112, the collimator assembly 1 may move in the axial direction of the through hole 112.

In an embodiment of the present disclosure, as shown in FIG. 2 to FIG. 5, the rotation of the collimator assembly 1 in a plane where the slit width is located may be adjusted by rotating corresponding second adjustment mechanisms in the through holes 112 of a first boss portion and a second boss portion by different angles. For example, when the collimator is horizontally placed, by rotating the bolt in the first boss portion and the bolt in the second boss portion by different angles, the collimator may be slightly rotated in the horizontal plane. In this way, a small-angle rotation of the collimator may be achieved. Alternatively, when the collimator is vertically placed, a fine deflection adjustment in the vertical direction may be achieved.

In an embodiment of the present disclosure, as shown in FIG. 2 to FIG. 5, the position adjustment portion 11 further includes a flat plate portion 114. The flat plate portion 114 is provided with a plurality of flat plate holes 1141 and an elongated gap 1142, the elongated gap 1142 corresponds to the collimator slit 125, and the flat plate holes 1141 respectively correspond to the adjustment holes 124. The boss portion 111 may be located at an end portion of the flat plate portion 114. Preferably, each of two ends of the flat plate portion 114 is provided with a boss portion 111. Since the flat plate portion 114 is provided with the flat plate holes 1141 corresponding to the adjustment holes 124, screw rods may penetrate the adjustment holes 124 and the flat plate holes 1141 to adjust the flat plate portion 114, the left collimator member 122, and the right collimator member 123 together. The flat plate portion 114 may include a left flat plate portion and a right flat plate portion, which correspond to the left collimator member 122 and the right collimator member 123 respectively. The elongated gap 1142 is formed between the left flat plate portion and the right flat plate portion to correspond to the collimator slit 125. Alternatively, in other embodiments, the flat plate portion 114 is formed as an integral flat plate, in which the elongated gap 1142 having a width greater than or equal to the width of the collimator slit is formed.

In an embodiment of the present disclosure, as shown in FIG. 2 to FIG. 5, the collimator includes a plurality of adjustment holes 124. Each of the adjustment holes 124 and the flat plate holes 1141 has a smooth inner surface; that is, neither the adjustment holes 124 nor the flat plate holes 1141 are threaded holes. The first adjustment member 121 penetrates the flat plate hole 1141 and the adjustment hole 124, and is fixed to the shielding box 13 through a nut.

In an embodiment of the present disclosure, as shown in FIG. 2 to FIG. 5, the left collimator member 122 includes a first protrusion 1221 at a position close to the collimator slit 125, and the right collimator member 123 includes a second protrusion 1231 at a position close to the collimator slit 125. The first protrusion 1221 and the second protrusion 1231 surround the collimator slit 125. Accordingly, the flat plate portion 114 has an opening for the first protrusion 1221 and the second protrusion 1231 to pass through.

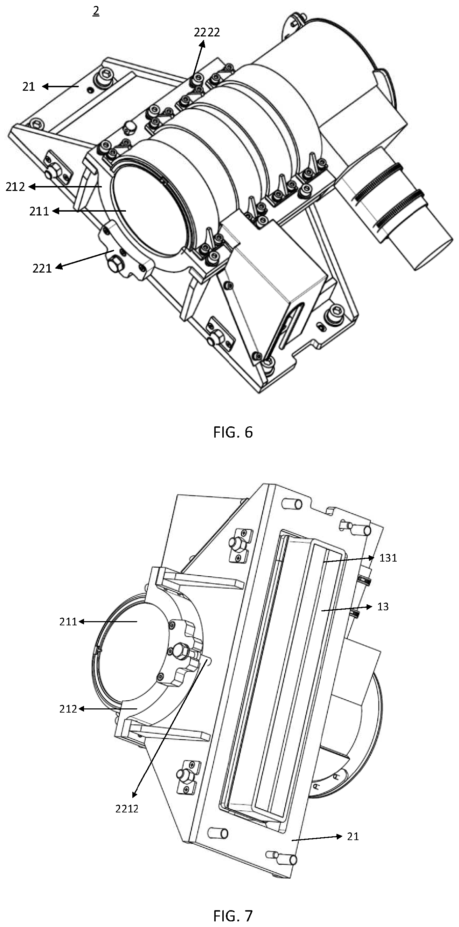

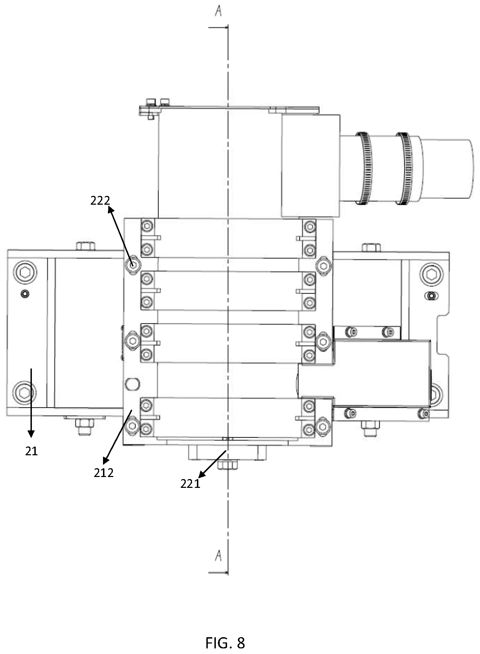

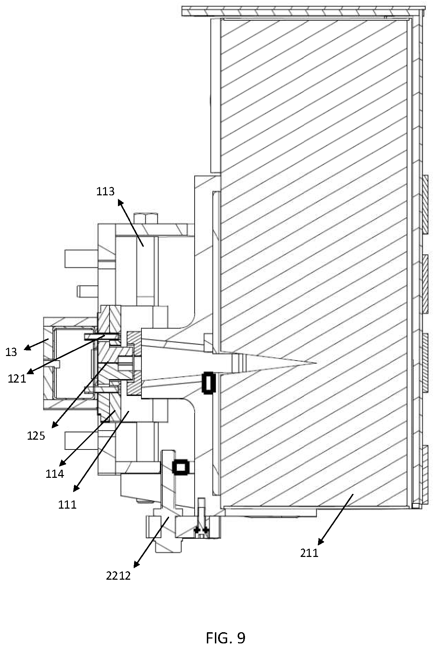

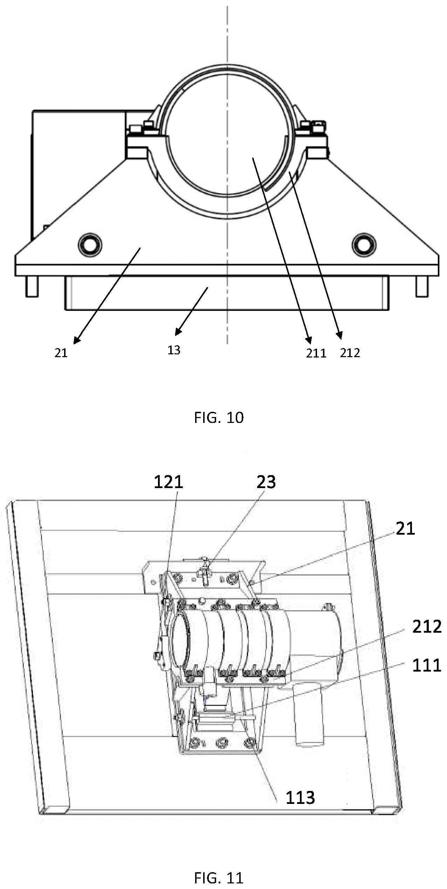

In the present disclosure, an X-ray source adjustment device 2 is further provided. FIG. 6 is a perspective view of an X-ray source adjustment device according to an exemplary embodiment of the present disclosure. FIG. 7 is a perspective view of an X-ray source adjustment device from another angle according to an exemplary embodiment of the present disclosure. FIG. 8 is a top view of an X-ray source adjustment device according to an exemplary embodiment of the present disclosure. FIG. 9 is a sectional view taken along line A-A shown in FIG. 8. FIG. 10 is a side view of an X-ray source adjustment device according to an exemplary embodiment of the present disclosure. FIG. 11 is a perspective view of an X-ray source base, vertically arranged and having an X-ray source adjustment device mounted thereon, according to an exemplary embodiment of the present disclosure.

As shown in FIG. 6 to FIG. 11, the X-ray source adjustment device 2 includes: a base 21, where an X-ray tube 211 and an X-ray tube bracket 212 are arranged on the base 21; an X-ray tube horizontal adjustment member 22, including a first horizontal adjustment member 221 and a plurality of second horizontal adjustment members 222, the second horizontal adjustment members 222 are located on the X-ray tube bracket 212, the first horizontal adjustment member 221 is used to connect the X-ray tube bracket 212 to the base 21, and the second horizontal adjustment members 222 are used to adjust a position of the X-ray tube 211 relative to the X-ray tube bracket 212; and the collimator assembly 1 described above, where the collimator assembly 1 is located on the base 21.

In an embodiment of the present disclosure, as shown in FIG. 6 to FIG. 11, the base 21 of the X-ray source is vertically arranged, the X-ray tube bracket 212 is arranged on the base 21, and the X-ray tube 211 is arranged on the X-ray tube bracket 212 and thus arranged on the base 21. On one hand, the first horizontal adjustment member 221 may connect the X-ray tube bracket 212 to the base 21; on the other hand, the first horizontal adjustment member 221 may adjust the position (preferably a horizontal position) of the X-ray tube bracket 212 relative to the base 21. The first adjustment member 121 includes a third adjustment member 2212 and a threaded hole that is provided on the X-ray tube bracket 212. For example, the third adjustment member 2212 may be a screw rod or a bolt. By rotating the screw rod or bolt, the X-ray tube bracket 212 may be mounted to the base 21. By turning the screw rod or bolt to loosen the screw rod or bolt, the X-ray tube bracket 212 is allowed to move in the horizontal direction, thereby adjusting the position of the X-ray tube bracket 212 in the horizontal direction.

In an embodiment of the present disclosure, as shown in FIG. 6 to FIG. 11, the second horizontal adjustment members 222 are provided on two sides of the X-ray tube 211, including a fourth adjustment member 2221 and an elongated adjustment hole, where the elongated adjustment hole is provided on the X-ray tube bracket 212. The fourth adjustment member 2221 penetrates the elongated adjustment hole to adjust a horizontal position of the X-ray tube 211 relative to the X-ray tube bracket 212. Specifically, the fourth adjustment member 2221 may include a bolt or a screw rod. For example, the third adjustment member 2212 may be a screw rod or a bolt. By rotating the screw rod or bolt, the X-ray tube 211 may be mounted to the X-ray tube bracket 212. Threaded elongated holes 2222 are provided on the bracket on two sides of the X-ray tube 211, and a longitudinal direction of the hole extends along the horizontal direction. The second horizontal adjustment member 222 penetrates the elongated hole 2222 to mount the X-ray tube 211 to the X-ray tube bracket 212, and also allow the X-ray tube 211 to move in the horizontal direction, thereby adjusting the position of the X-ray tube 211 in the horizontal direction.

As shown in FIG. 9, an axis of the X-ray tube 211 is perpendicular to a slit width direction of the collimator assembly 1. Rays emitted from the X-ray tube 211 penetrate the collimator slit 125 and continue to travel forward. As shown in the drawings, the slit width is associated with a distance from the collimator assembly 1 to the X-ray tube 211, and the closer the collimator assembly is to the X-ray tube 211, the narrower the collimator slit will be.

In an embodiment of the present disclosure, as shown in FIG. 11, the X-ray source adjustment device 2 may further include a vertical adjustment member 23. The vertical adjustment member 23 includes a screw rod and a nut. The screw rod penetrating the X-ray tube bracket 212 is connected to the base 21 through the nut, so as to adjust a vertical position of the X-ray tube 211 relative to the base 21.

In an embodiment of the present disclosure, as shown in FIG. 11, the base 21 of the X-ray source may be arranged in a plane perpendicular to the horizontal plane. That is, the base 21 may be arranged in a vertical plane.

In the present disclosure, an X-ray imaging system is further provided, including the X-ray source adjustment device 2 described above.

It should also be understood that when a layer is referred to as being "on" another layer, it may be directly on the another layer, or intervening layers may also be present. In contrast, when an element is referred to as being "directly on", "directly connected to", or "directly coupled to" another element or layer, no intervening element or layer is present. The term "and/or" as used herein includes any and all combinations of one or more of the associated listed items. Known functions or structures may not be described in detail for brevity and/or clarity.

As used herein, terms such as "first", "second", and the like are used to describe various members, components, regions, layers, and/or portions. However, it is apparent that the members, components, regions, layers, and/or portions should not be limited by these terms. These terms are used only to distinguish one member, component, region, layer, or portion from another member, component, region, layer, or portion. Therefore, a first member, component, region, layer, or portion to be described may also be referred to as a second member, component, region, layer, or portion without departing from the scope of the present disclosure.

The foregoing description of exemplary embodiments of the present disclosure is presented only for purposes of illustration and description, and is not intended to be exhaustive or to limit the present disclosure to the precise forms disclosed. Many modifications and variations are possible in light of the above teachings. The embodiments are selected and described to explain the principles and practical applications of the present disclosure, so that others skilled in the art can use the present disclosure and various embodiments, with various modifications suitable for the particular use contemplated. Alternative embodiments will become apparent to those skilled in the art to which the present disclosure pertains without departing from the spirit and scope of the present disclosure. Therefore, the scope of the present disclosure is defined by the appended claims rather than by the foregoing description and the exemplary embodiments described therein.

## Claims

1. A collimator assembly, comprising:
at least one position adjustment portion located at an end portion of the collimator assembly and configured to adjust a position of the collimator assembly;
a collimator body portion, wherein the at least one position adjustment portion is located on the collimator body portion, the collimator body portion comprises a first adjustment member, a left collimator member, a right collimator member, and adjustment holes provided on the left collimator member and the right collimator member, the left collimator member and the right collimator member are arranged side by side with a collimator slit between the left collimator member and the right collimator member, and the adjustment member penetrates the adjustment hole to adjust a collimator slit width; and
a shielding box fixed to a side of the collimator body portion opposite to the at least one position adjustment portion, wherein each of a top portion of the shielding box and a bottom portion of the shielding box is provided with a gap corresponding to the collimator slit.

2. The collimator assembly according to claim 1, wherein the position adjustment portion comprises a boss portion, a through hole is provided in the boss portion, and at least a section of an inner surface of the through hole is provided with a thread.

3. The collimator assembly according to claim 2, wherein the collimator assembly comprises a first boss portion and a second boss portion, each through hole is provided with a second adjustment member having a thread, and the second adjustment member is configured to fix the collimator assembly and adjust a position of the collimator assembly in an axial direction of the through hole.

4. The collimator assembly according to claim 2, wherein the collimator assembly comprises a first boss portion and a second boss portion, and a rotation of the collimator assembly in a plane where the slit width is located is adjustable by rotating corresponding second adjustment mechanisms in the through holes of the first boss portion and the second boss portion by different angles.

5. The collimator assembly according to claim 4, wherein each of the first adjustment member and the second adjustment member is a screw rod.

6. The collimator assembly according to any one of claims 1 to 5, wherein the collimator slit width is in a range of 0.6 mm to 1.2 mm.

7. The collimator assembly according to any one of claims 1 to 5, wherein the at least one position adjustment portion further comprises a flat plate portion, the flat plate portion is provided with a plurality of flat plate holes and an elongated gap, the elongated gap is in correspondence with the collimator slit, and the plurality of flat plate holes are in one-to-one correspondence with the adjustment holes provided on the collimator body portion respectively.

8. The collimator assembly according to claim 7, wherein each of the adjustment holes and each of the plurality of flat plate holes has a smooth inner surface, and the first adjustment member is fixed to the shielding box by penetrating the flat plate hole and the adjustment hole.

9. The collimator assembly according to any one of claims 1 to 5, wherein the left collimator member comprises a first protrusion at a position close to the collimator slit, the right collimator member comprises a second protrusion at a position close to the collimator slit, and the collimator slit is surrounded by the first protrusion and the second protrusion.

10. An X-ray source adjustment device, comprising:
a base, wherein an X-ray tube and an X-ray tube bracket are arranged on the base;
an X-ray tube horizontal adjustment member, comprising a first horizontal adjustment member and a second horizontal adjustment member, wherein the second horizontal adjustment member is located on the X-ray tube bracket, the first horizontal adjustment member is configured to adjust a position of the X-ray tube bracket relative to the base, and the second horizontal adjustment member is configured to adjust a position of the X-ray tube relative to the X-ray tube bracket; and
the collimator assembly according to any one of the preceding claims, wherein the collimator assembly is located on the base.

11. The X-ray source adjustment device according to claim 10, wherein the first horizontal adjustment member is arranged on the X-ray tube bracket, the first horizontal adjustment member comprises a third adjustment member and a threaded hole, the threaded hole is provided in the X-ray tube bracket, and the third adjustment member is fixed to the base by penetrating the threaded hole.

12. The X-ray source adjustment device according to claim 10, wherein a plurality of second horizontal adjustment members are provided on two sides of the X-ray tube respectively, the second horizontal adjustment member comprises a fourth adjustment member and an elongated adjustment hole, the elongated adjustment hole is provided on the X-ray tube bracket, and the fourth adjustment member penetrates the elongated adjustment hole to adjust a horizontal position of the X-ray tube relative to the X-ray tube bracket.

13. The X-ray source adjustment device according to claim 11, wherein a longitudinal direction of the elongated adjustment hole is parallel to an axis of the X-ray tube.

14. The X-ray source adjustment device according to claim 11, wherein an axis of the X-ray tube is perpendicular to a slit width direction of the collimator assembly.

15. The X-ray source adjustment device according to claim 11, wherein rays emitted from the X-ray tube penetrate the collimator slit, the slit width is in association with a distance from the collimator assembly to the X-ray tube, and the collimator slit is narrower as the collimator assembly gets closer to the X-ray tube.

16. The X-ray source adjustment device according to claim 15, further comprising a vertical adjustment member, wherein the vertical adjustment member comprises a screw rod and a nut, the screw rod penetrating the X-ray tube bracket is connected to the base through the nut, and is configured to adjust a vertical position of the X-ray tube relative to the base.

17. The X-ray source adjustment device according to claim 11, wherein the base is arranged in a plane perpendicular to a horizontal plane.

18. An X-ray imaging system, comprising the X-ray source adjustment device according to any one of the preceding claims.
